# EUROPEAN PATENT APPLICATION

(11) **EP 2 641 915 A1**
(43) Date of publication of application: **25.09.2013**
(21) Application number: 13159160.4
(22) Date of filing: 14.03.2013
(51) Int. Cl.: C07K 16/28

(54) **Antibodies to the chemokine receptor XCR1**

(30) Priority: 15.03.2012 EP 12001756
(71) Applicant: Kroczek, Richard, 14055 Berlin (DE)
(72) Inventor: Kroczek, Richard, 14055 Berlin (DE)
(74) Representative: Bösl, Raphael Konrad

(57) **Abstract**

The present invention relates to an antibody binding to murine XCR1. Furthermore, the present invention relates to a nucleic acid molecule encoding for said antibody, a vector comprising said nucleic acid molecule and a host cell comprising said vector. The present invention further embraces a method for producing the antibody. Moreover, the present invention claims the use of the antibody and a kit for detecting murine XCR1⁺ cells and/or separating murine XCR1⁺ cells from other cells.

SEQUENCE LISTING

<110>  Kroczek, Richard

<120>  Antibodies to the chemokine receptor XCR1

<130>  K68115EP1

<160>  3

<170>  PatentIn version 3.5

<210>  1
<211>  338
<212>  PRT
<213>  Mus musculus

<400>  1

Met Asp Ser Glu Ser Asp Ala Leu Ser Ile Pro Ala Ser Arg Val Gln
1               5                   10                  15

Met Glu Ser Ser Thr Ala Phe Tyr Asp Tyr His Asp Lys Leu Ser Leu
            20                  25                  30

Leu Cys Glu Asn Asn Val Ile Phe Phe Ser Thr Ile Ser Thr Ile Val
        35                  40                  45

Leu Tyr Ser Leu Val Phe Leu Leu Ser Leu Val Gly Asn Ser Leu Val
    50                  55                  60

Leu Trp Val Leu Val Lys Tyr Glu Asn Leu Glu Ser Leu Thr Asn Ile
65                  70                  75                  80

Phe Ile Leu Asn Leu Cys Leu Ser Asp Leu Met Phe Ser Cys Leu Leu
                85                  90                  95

Pro Val Leu Ile Ser Ala Gln Trp Ser Trp Phe Leu Gly Asp Phe Phe
            100                 105                 110

Cys Lys Phe Phe Asn Met Ile Phe Gly Ile Ser Leu Tyr Ser Ser Ile
        115                 120                 125

Phe Phe Leu Thr Ile Met Thr Ile His Arg Tyr Leu Ser Val Val Ser
    130                 135                 140

Pro Ile Ser Thr Leu Gly Ile His Thr Leu Arg Cys Arg Val Leu Val
145                 150                 155                 160

Thr Ser Cys Val Trp Ala Ala Ser Ile Leu Phe Ser Ile Pro Asp Ala
                165                 170                 175

Val Phe His Lys Val Ile Ser Leu Asn Cys Lys Tyr Ser Glu His His
            180                 185                 190

Gly Phe Leu Ala Ser Val Tyr Gln His Asn Ile Phe Phe Leu Leu Ser
        195                 200                 205

Met Gly Ile Ile Leu Phe Cys Tyr Val Gln Ile Leu Arg Thr Leu Phe
    210                 215                 220

Arg Thr Arg Ser Arg Gln Arg His Arg Thr Val Arg Leu Ile Phe Thr
225                 230                 235                 240

Val Val Val Ala Tyr Phe Leu Ser Trp Ala Pro Tyr Asn Leu Thr Leu
                245                 250                 255

Phe Leu Lys Thr Gly Ile Ile Gln Gln Ser Cys Glu Ser Leu Gln Gln
            260                 265                 270

Leu Asp Ile Ala Met Ile Ile Cys Arg His Leu Ala Phe Ser His Cys
        275                 280                 285

Cys Phe Asn Pro Val Leu Tyr Val Phe Val Gly Ile Lys Phe Arg Arg
    290                 295                 300

His Leu Lys His Leu Phe Gln Gln Val Trp Leu Cys Arg Lys Thr Ser
305                 310                 315                 320

Ser Thr Val Pro Cys Ser Pro Gly Thr Phe Thr Tyr Glu Gly Pro Ser
                325                 330                 335

Phe Tyr

<210>  2
<211>  219
<212>  PRT
<213>  Mus musculus

<400>  2

Asp Val Val Val Thr Gln Thr Pro Leu Ser Leu Pro Val Ser Leu Gly
1               5                   10                  15

Asp Pro Ala Ser Ile Ser Cys Lys Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Ile Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Asn
                85                  90                  95

Thr His Val Pro Tyr Thr Phe Gly Gly Gly Thr Lys Leu Glu Ile Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser
        195                 200                 205

Pro Ile Val Lys Ser Phe Asn Arg Asn Glu Cys
    210                 215

<210>  3
<211>  151
<212>  PRT
<213>  Mus musculus

<400>  3

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Ile His Trp Met Lys Gln Arg Pro Gly Gln Gly Leu Glu Trp Ile
        35                  40                  45

Gly Met Ile His Pro Asn Ser Asp Asn Thr Lys Tyr Asn Glu Lys Phe
    50                  55                  60

Lys Ala Lys Ala Ile Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Phe Ala Asn Asp Gly Ala Tyr Trp Gly Gln Gly Thr Leu Val
            100                 105                 110

Thr Val Ser Ala Ala Lys Thr Thr Pro Pro Ser Val Tyr Pro Leu Ala
        115                 120                 125

Pro Gly Cys Gly Asp Thr Thr Gly Ser Ser Val Thr Leu Gly Cys Leu
    130                 135                 140

Val Lys Gly Tyr Phe Pro Glu
145                 150

## Description

### TECHNICAL FIELD

Murine XCL1, also known as murine lymphotactin, is an important chemokine attracting lymphocytes chemotactically by binding to a chemokine receptor called murine XCR1. XCL1 is found on activated thymic and peripheral blood CD8⁺ T cells and is, thus, found in spleen, thymus, intestine and peripheral blood leukocytes of mice. It may also be found in lung, prostate gland and ovary of mice.

XCL1 has been originally cloned as lymphotactin/ATAC/SCM-1 (Kelner et al., 1994; Müller et al., 1995; Yoshida et al., 1995). The receptor for murine XCL1, designated murine XCR1, has been identified in the human system by the group of Yoshie (Yoshida et al. 1998), later the same group cloned the murine XCR1 (Yoshida et al., 1999).

Recently, we determined that XCR1 is exclusively expressed on CD8⁺ CD11c⁺ MHC-class II^{high} antigen cross-presenting dendritic cells (DCs) in the mouse (Domer et al., 2009). Later, we and others identified XCR1 expression on DCs in the human (Bachem et al. 2010, Crozat et al. 2010). Until now, the expression of XCR1 could only be detected using transgenic reporter mice (B6.129P2-Xcrl^{tm1Dgen}/J, The Jackson Laboratories, B6.XCR1-LacZ, Domer et al., 2009), in which the XCR1 gene has been replaced with the enzyme β-galactosidase by homologous recombination. This enzyme can process the nonfluorescent substrate fluorescein-di-β-D-galactopyranoside (FDG) to fluorescein, which can be detected by flow cytometry, the cells, however, are damaged in this assay. Alternatively, XCR1 could be detected at the mRNA level using qualitative or quantitative PCR (Domer et al. 2009).

In the view of the above, the methods for detecting XCR1 known in the art, i.e., detecting XCR1 by means of transgenic reporter mice and detecting XCR1 at the mRNA level by means of qualitative or quantitative PCR, do neither allow quantifying the number of murine XCR1⁺ cells in a blood sample nor separating XCR1⁺ cells from other cells. Furthermore, both aforementioned methods are comparably laborious and require special skills of the operator who must be able and accredited to work with transgenic animals or with chemically and biologically instable mRNA, which is not easy to handle in practice.

Therefore, there is still a need for a method for quantifying the number of murine XCR1⁺ cells in a blood sample and for separating XCR1⁺ cells from other cells.

### SUMMARY

Surprisingly, an antibody binding to murine XCR1 was generated, which is suitable for detecting murine XCR1⁺ cells and/or for separating murine XCR1⁺ cells from other cells.

Therefore, in a first aspect, the present invention relates to an antibody binding to murine XCR1, in particular to a monoclonal antibody.

In one embodiment of the first aspect, said antibody is specific for dendritic cells, in particular murine dendritic cells, and said antibody preferably does not block chemotaxis of XCR1⁺ dendritic cells to the murine chemokine ligand XCL1. Said antibody preferably is also a murine antibody.

In another embodiment of the first aspect, said antibody is conjugated with at least one labeling moiety or with at least one solid support, preferably a bead, in particular a magnetic bead or an affinity chromatography bead.

In a second aspect, the invention relates to a nucleic acid molecule encoding the described antibody.

In a third aspect, the invention relates to a vector comprising said nucleic acid.

In a forth aspect, the inventions relates to a host cell comprising said vector.

In one embodiment of the forth aspect, said host cell is a hybridoma cell.

In a fifth aspect, the invention relates to a method for producing said antibody, said method comprising:
(i) culturing said host cell under conditions that allow said host cell to express said antibody, and
(ii) recovering the antibody from said culture.

In a sixth aspect, the invention relates to a method for
(i) detecting murine XCR1⁺ cells, in particular by means of cytometry or immunohistology, and/or
(ii) separating murine XCR1⁺ cells from other cells, in particular by means of cell sorting, with the use of said antibody.

In a seventh aspect, the invention relates to a kit for detecting murine XCR1⁺ cells, said kit comprises said antibody, said nucleic acid, said vector and/or said host cell, and preferably further comprises a user manual.

In an eighth aspect, the invention relates to a kit for separating murine XCR1⁺ cells from other cells, said kit comprises said antibody, said nucleic acid, said vector and/or said host cell, and preferably further comprising a user manual.

### DESCRIPTION OF EMBODIMENTS

According to the first aspect, the present invention relates to an antibody binding to murine XCR1.

As used in the context of the present invention, the term "antibody" may be understood in the broadest sense as any type of antibody, antibody fragment, antibody mutant or antibody mimetic.

Exemplarily, the antibody of the present invention may be an immunoglobulin A (IgA), immunoglobulin D (IgD), immunoglobulin E (IgE), immunoglobulin G (IgG), immunoglobulin M (IgM), immunoglobulin Y (IgY) or immunoglobulin W (IgW). Preferably, the antibody is an IgA, IgG or IgD. More preferably, the antibody is an IgG. Even more preferably, the antibody is an IgG2. Most preferably, the antibody is an IgG2b. However, it will be apparent that the type of antibody may be altered by biotechnological means by cloning the gene encoding for the antigen-binding domains of the antibody of the present invention into a common gene construct encoding for any other antibody type.

As used herein, the term "antibody fragment" may be understood in the broadest sense as any fragment of an antibody that still bears binding affinity to murine XCR1. Exemplarily, the antibody fragment may be a fragment antigen binding (Fab fragment), a truncated antibody comprising one or both complementarity determining region(s) (CDR(s)) or the variable fragment (Fv) of an antibody. The antibody fragments may be of natural origin, of gene technologic origin and/or of synthetical origin.

As used herein, the term "antibody mimetic" may be understood in the broadest sense as organic compounds that, like antibodies, can specifically bind antigens and that typically have a molecular mass in a range of from approximately 3 kDa to approximately 25 kDa. Antibody mimetics may be, e.g., affibody molecules (affibodies), affilins, affitins, anticalins, avimers, DARPins, Fynomers, Kunitz domain peptides, single-domain antibodies (e.g., V_{H}H antibodies or V_{NAR} antibodies, nanobodies), monobodies, diabodies, triabodies, flexibodies and tandabs. The antibody mimetics may be of natural origin, of gene technologic origin and/or of synthetical origin. Optionally, the antibody may also be a CovX-body. Optionally, the antibody may also be a cameloid species antibody.

It will be understood that the term "antibody" as used in the context of the present invention also encompasses the salts thereof. The antibody, independent on its chemical nature, may optionally be dissolved in any medium suitable for storing said antibody such as, e.g., water, an aqueous buffer (e.g., a Hepes, Tris, or phosphate buffer), blood, serum, plasma, lymph or an organic solvent (e.g., dimethyl sulfoxide (DMSO), dimethylformide (DMF)). It will further be understood that the antibody may be stored at conditions suitable for it, such as, e.g., in solution at room temperature, in solution in a cooled environment (e.g., at 4°C), in frozen (at -20°C), deep-frozen (e.g., -80°C, -196°C), freeze-dried and/or dry state depending on the chemical nature of the antibody.

Preferably, the binding between the antibody and the antigen is a non-covalent binding. Preferably, the binding affinity of the antibody to its antigen has a dissociation constant (Kd) of less than 1 µM, less than 500 nM, less than 200 nM, less than 100 nM, less than 50 nM, less than 40 nM, less than 30 nM or even less than 20 nM.

Preferably, the antibody has a molecular weight of more than 3 kDa, more than 5 kDa, more than 10 kDa, more than 20 kDa, more than 30 kDa, more than 50 kDa, more than 100 kDa, more than 110 kDa, more than 120 kDa, more than 130 kDa or even more than 135 kDa. Most preferably, the molecular weight of the antibody is between 135 kDa and 170 kDa.

The antibody of the present invention may be of any species or origin. It may bind to any epitope(s) comprised in the murine XCR1 polypeptide (e.g., linear epitope(s), structural epitope(s), primary epitope(s), secondary epitope(s)), including its posttranslational modifications. The epitope may be accessible by the antibody in the natural configuration of the murine XCR1 or may be a hidden epitope (particularly detectable in denaturized polypeptides). In general, the antibody may recognize the naturally folded murine XCR1 or a domain or fragment thereof and/or may recognize the denaturized murine XCR1 or a domain or fragment thereof. Preferably, the epitope is accessible in the natural configuration of the murine XCR1. The antibody may be of natural origin, of gene technologic origin and/or of synthetical origin.

The antibody may be isolated or may be comprised in a serum or in a culture broth. As used in this context, the term "isolated" may be understood is a way that the antibody constitutes at least 50 %(w/w), at least 60 %(w/w), at least 70 %(w/w), at least 80 %(w/w), at least 90 %(w/w), at least 95 %(w/w), or even 100 %(w/w) of the total antibody content. Preferably, an isolated antibody may constitute for at least 20 %(w/w), at least 40 %(w/w), at least 50 %(w/w), at least 60 %(w/w), at least 70 %(w/w), at least 80 %(w/w), at least 90 %(w/w) or even 100 %(w/w) of the total protein content. It will, however, be understood that also an isolated antibody may optionally be dissolved in a liquid medium as described above.

Preferably, the antibody of the present invention is specific for murine XCR1. However, alternatively, the antibody may also bind other targets bearing similar amino acid sequences of other, in particular of evolutionary related, polypeptides.

As used herein, an antibody specific for murine XCR1 binds to cells that express murine XCR1 and expose it on their surface (XCR1⁺ cells), whereas it does not or nearly not bind to cells that do not express murine XCR1 (XCR1⁻ cells). Preferably, the ratio of antibody binding to XCR1⁺ cells to antibody binding to XCR1⁻ cells is at least 5:1, at least 10:1, at least 50:1, at least 100:1, at least 1,000:1 or at least 10,000:1. Herein, the cells may preferably be dendritic cells.

As used throughout the present invention, the terms "XCR1⁺ cell" and "XCR1 positive cell" refer to any cell bearing murine XCR1 on the cellular surface, in particular bearing murine XCR1 on the cellular surface in a way detectable by the antibody of the present invention. In general, as used herein and as generally understood in the art, the designation "+" means that a factor (e.g., depending on the context, a polypeptide, a nucleic acid molecule encoding said polypeptide and/or a vector comprising said nucleic acid molecule) is present, whereas "-" refers to the substantial absence thereof.

Likewise, the specificity may be determined by determining the binding to murine XCR1-coated beads (e.g., by flow cytometry).

Moreover, as used herein, an antibody specific for murine XCR1 binds to murine XCR1 with a higher affinity than to any other polypeptide that does not comprise the epitope recognized by the antibody, typically an amino acid sequence of at least eight consecutive amino acids as comprised by murine XCR1. Preferably, this higher affinity is at least five times higher , at least ten times higher, at least 20 time higher, at least 50 time higher, at least 100 time higher, at least 1,000 times higher, at least 10,000 times higher or even more than 10,000 times higher.

The specificity may be determined by any means known in the art such as, e.g., a binding assay (e.g., by Affimetrix Technology, by fluorescence correlation spectroscopy (FCS), by fluorescence cross-correlation spectroscopy (FCCS)) which are also commercially available (e.g., by Affimetrix, Inc., Santa Clara, California, U.S.A.; Intana Bioscience GmbH, Munich, Germany). Further methods to determine the specificity are exemplified in the example section below.

The antibody of the present invention may be obtained from any biological or synthetic source known in the art to be suitable for producing antibodies. Exemplarily, the antibody may be obtained from an immunized animal (e.g., from the blood, from the serum, from the plasma, from the lymph) or may be obtained from cell or tissue culture. The antibody may be a polyclonal or a monoclonal antibody (mAb).

XCR1 is a chemokine receptor of the "C" sub-family of chemokine receptors and is a G protein-coupled receptor. The full-length murine XCR1 bears seven transmembrane domains and numerous conserved amino acids and is able to transduce a signal by increasing the intracellular calcium ions level.

As used throughout the present invention, the terms "XCR1", "chemokine XC receptor 1", "XCL1 receptor", "lymphotactin receptor", "ATAC receptor", "SCM-1 receptor" and "GPR5" and may be understood exchangeably as the receptor protein or a functional derivative thereof having a sequence homology of at least 80 %, at least 85 %, at least 90 % or between 90 and 100 % of murine XCR1 or a peptide of at least seven, at least eight, at least nine, at least ten, at least 15, at least 20, at least 30, at least 50 or at least 100 consecutive amino acids of the sequence of a murine XCR1 polypeptide or a derivative thereof, wherein said peptide may serve as an epitope and, therefore, as specific antigen of the antibody of the present invention.

As used herein, murine XCR1 has the sequence homology of at least 80 %, at least 85 %, at least 90 % or between 90 and 100 % the polypeptide sequence of SEQ ID NO:1:

MDSESDALSIPASRVQMESSTAFYDYHDKLSLLCENNVIFFSTISTIVLYSLVFLLSL
VGNSLVLWVLVKYENLESLTNIFILNLCLSDLMFSCLLPVLISAQWSWFLGDFFCK
FFNMIFGISLYSSIFFLTIMTIHRYLSVVSPISTLGIHTLRCRVLVTSCVWAASILFSIP
DAVFHKVISLNCKYSEHHGFLASVYQHNIFFLLSMGIILFCYVQILRTLFRTRSRQR
HRTVRLIFTVVVAYFLSWAPYNLTLFLKTGIIQQSCESLQQLDIAMIICRHLAFSHCC
FNPVLYVFVGIKFRRHLKHLFQQVWLCRKTSSTVPCSPGTFTYEGPSFY

Murine XCR1 is also described and cloned in the art (cf., e.g., Yoshida et al., 1998; Yoshida et al., 1999).

XCR1 binds to murine XCL1 and human XCL1 and XCL2. As used herein, the term "XCL1" may be understood interchangeably with "chemokine XC 1", "lymphotactin", "ATAC", "SCM-1" and "chemokine (C motif) ligand" as described in the art (Kelner et al., 1994; Müller et al.; 1995, Yoshida et al., 1995). XCL1 is a small cytokine belonging to the XC chemokine family. Typically, XCL1 is found in high levels in activated immune cells in the spleen, thymus, intestine and peripheral blood leukocytes, and at lower levels in lung, prostate gland and ovary. It may be found in activated CD8+ T cells. Murine XCL1 is closely related to human XCL1 and XCL2. XCL1 is able to induce chemotactic function by binding to the chemokine receptor XCR1.

In a preferred embodiment, said antibody is a monoclonal antibody.

Monoclonal antibodies are well-known to those skilled in the art. Monoclonal antibodies of one kind all bear a single specificity, i.e. recognize and bind to a single epitope. Typically, hybridoma cells are produced by hybridoma cells or bacteria. However, all other cells and cell-free expression systems are in general also suitable for producing monoclonal antibodies.

The antibody of the present invention may bind to murine XCR1 in any molecular context. Exemplarily, murine XCR1 or a derivative or peptide thereof may be localized on a cell surface, may be soluble, may be immobilized on a surface (e.g., a microarray surface (e.g., a grass surface, a hydrogen surface), a bead surface (e.g., a magnetic bead surface), an affinity chromatography matrix surface)). Preferably, murine XCR1 is localized on a cell surface, in particular a surface of a dendritic cell (DC).

Typically, murine XCR1 is specifically expressed on cross-presenting dendritic cells, antigen cross-presenting dendritic cells, even more in particular on CD8⁺ CD11c⁺ antigen cross-presenting murine dendritic cells, most particularly on CD8⁺ CD11c⁺ MHC-class II^{high} antigen cross-presenting dendritic cells (Domer et al., 2009).

In a preferred embodiment, the antibody of the present invention is a monoclonal antibody to murine XCR1, in particular a murine monoclonal antibody to murine XCR1.

Therefore, in a preferred embodiment, the antibody of the present invention is specific for dendritic cells, in particular murine dendritic cells.

In this context, the DCs are preferably antigen cross-presenting DCs, even preferably CD8⁺ CD11c⁺ antigen cross-presenting DCs, particularly CD8⁺ CD11c⁺ MHC-class II^{high} antigen cross-presenting DCs.

In the art, it is known that DC populations can be subdivided into "conventional" DCs (cDCs, encompassing CD8⁺ DCs, CD4⁺ DCs, and double negative DCs) and "plasmacytoid" DCs (Hashimoto et al. 2011).

Accordingly, the antibody of the present invention is specific for cDCs (i.e., subsets of CD8⁺ DCs, CD4⁺ DCs, and double negative DCs)

The antibody may or may not have an impact on chemotaxis of XCR1⁺ DCs to the murine chemokine ligand XCL1, i.e., it may or may not influence the binding equilibrium between unbound the unbound ligands, i.e., murine XCR1 (typically localized on DC cell surfaces) and murine XCL1 and the murine XCR1-XCL1 complex. The XCR1⁺ DCs as used herein are preferably murine XCR1⁺ DCs. Therefore, the antibody may or may not be a competitive, uncompetitive or noncompetitive (e.g., allosteric) inhibitor of the murine XCR1-XCL1 binding or a competitive, uncompetitive or noncompetitive (e.g., allosteric) activator of the murine XCR1-XCL1 binding. Preferably, the antibody does not bear inhibitory activity regarding the murine XCR1-XCL1 binding.

Accordingly, in a preferred embodiment, the antibody of the present invention does not block chemotaxis of XCR1⁺ DCs to the murine chemokine ligand XCL1.

Further, the antibody itself may or may not have activator or inhibitory effect on its binding partner, i.e., in particular murine XCR1. The antibody may bear a sequence originating from any species suitable of generating an antibody. Preferably, the antibody is a mammalian antibody.

The antibody may or may not have neutralizing activity on murine XCR1, murine XCR1-coated solid support(s) and/or XCR1⁺ cells.

In a more preferred embodiment, said antibody is a murine antibody.

Herein, the term "murine antibody" should not be understood in a way that the antibody molecule itself necessarily has to originate from a mouse, but rather in a way that it bears a sequence originating from mouse or a sequence having at least 80 % sequence identity, at least 85 % sequence identity, at least 90 % sequence identity, at least 95 % sequence identity or even 100 % sequence identity to the respective sequence of antibodies or fragments thereof of the mouse. Throughout the present invention, the terms "of the mouse", "originating from the mouse" and "murine" may be understood interchangeably where appropriate. Exemplarily, a murine antibody may recombinantly also be produced by another species such as, e.g., by a bacterium.

A specifically preferred murine monoclonal antibody contains the kappa light chain of SEQ ID NO: 2: 
DVVVTQTPLSLPVSLGDPASISCKSSQSLVHSNGNTYLHWYLQKPGQSPKLLIYKISNRFSGVPDR
FSGSGSGTDFTLKISRVEAEDLGVYFCSQNTHVPYTFGGGTKLEIKRADAAPTVSIFPPSSEQLTS
GGASVVCFLNNFYPKDINVKWKIDGSERQNGVLNSWTDQDSKDSTYSMSSTLTLTKDEYERHNSYT
CEATHKTSTSPIVKSFNRNEC
 wherein bold italics represent the variable region, bold italics underlined the CDRs 1-3 and normal letters the light chain constant region according to the IMGT numbering (IMGT^{®}, the international ImMunoGeneTics information system^{®}; http://www.imgt.org). Consequently, alternatively preferred murine monoclonal antibodies contain either the variable region (sequence in bold italics) or the CDRs 1-3 (sequences in bold italics underlined).

Another specifically preferred murine monoclonal antibody contains the IgG2b heavy chain of SEQ ID NO: 3: 
QVQLQQPGAELVKPGASVKLSCKASGYTFTNYWIHWMKQRPGQGLEWIGMIHPNSDNTKYNEKFKA
KAILTVDKSSSTAYMQLSSLTSEDSAVYYCARFANDGAYWGQGTLVTVSAAKTTPPSVYPLAPGCG
DTTGSSVTLGCLVKGYFPE...
 wherein again bold italics represent the variable region, bold italics underlined the CDRs 1-3 and normal letters in this case the beginning of the heavy chain constant regions according to the IMGT numbering. Consequently, alternatively preferred murine monoclonal antibodies contain either the variable region (sequence in bold italics) or the CDRs 1-3 (sequences in bold italics underlined).

Another specifically preferred murine monoclonal antibody contains (a) the variable region of the kappa light chain and the variable region of the IgG2b heavy chain as shown in SEQ ID NOs: 2 and 3, or (b) the CDRs 1-3 of both, the kappa light chain and the IgG2b heavy chain as shown in SEQ ID NOs. 2 and 3.

A particularly preferred murine monoclonal antibody, named MARX10, contains the variable region of the kappa light chain as shown in SEQ ID NO: 2 and the variable region of the IgG2b heavy chain as shown in SEQ ID NO: 3.

The antibody may be a plain, i.e., non-conjugated, antibody or may be an antibody conjugated with one or more non-peptidic or peptidic moiety/moieties.

In a preferred embodiment, the antibody according to the present invention is conjugated with at least one labeling moiety.

As used in the context of the present invention, the term "labeling moiety" may be understood in the broadest sense as any moiety that can be detected, in particular by a molecular imaging method.

Exemplarily, the labeling moiety may be a fluorescence label such as, e.g., a fluorescent polypeptide (e.g., cyan fluorescent protein (CFP), green fluorescent protein (GFP) or yellow fluorescent protein (YFP), red fluorescent protein (RFP), mCherry, etc.), a small-molecule dye (e.g., a Cy dye (e.g., Cy3, Cy5, Cy5.5, Cy 7), an Alexa dye (e.g., Alexa Fluor 488, Alexa Fluor 546, Alexa Fluor 647, Alexa Fluor 680, Alexa Fluor 750), a Visen dye (e.g. VivoTag680, VivoTag750), an S dye (e.g., S0387), a DyLight fluorophore (e.g., DyLight 750, DyLight 800), an IRDye (e.g., IRDye 680, IRDye 800), a fluorescein dye (e.g., fluorescein, carboxyfluorescein, fluorescein isothiocyanate (FITC)), a rhodamine dye (e.g., rhodamine, tetramethylrhodamine (TAMRA)) or a HOECHST dye), a quantum dot, a rare earth element or a combination of two or more thereof. However, preferably, the CPPs are not fluorescently stained.

Alternatively or additionally, the labeling moiety may be spin label such as, e.g., ²H or ¹³C. Alternatively or additionally, the labeling moiety may be radioactive label such as, e.g., ³H ¹⁴C, ¹²³I ¹²⁴I ¹³¹I, ³²P. Alternatively or additionally, the labeling moiety may be metal atom, metal ion or metal bead (e.g., a gold bead).

The labelling moiety may also be a lipid or alipidoid, a small molecule dye (e.g., a luminescent dye, an UV/Vis dye (e.g., a p-nitrophenyl moiety, Coomassie Brilliant Blue G-250), a binding moiety (e.g., biotin, methotrexate, glycocorticoids or moieties comprising (e.g., an active ester, an isothiocyanate, a maleimid, an N-hydroxysuccinimidyl ester, a glutaraldehyde derivative, a carbodiimide derivative or a combination thereof), an insoluble and/or soluble polymer (e.g., polyethylene glycol (PEG), hydroxypropyl methacrylate (HPMA), polyethylene imine (PEI)), another antibody (e.g., a Fab fragment, a single chain antibody, a diabody, a triabody, a flexibody, a tandab), a peptide (e.g., a cell-penetrating peptide (CPP), a protein transduction domain (PTD), a signal transduction peptide), an enzyme label (e.g., penicillinase, horseradish peroxidase, alkaline phosphatase), a micro- or nanobead (e.g., a functionalized silica bead, a polysaccharide-based bead), a polymersome, a micelle and/or a liposome.

In a preferred embodiment, the antibody according to the present invention is conjugated with at least one solid support.

As used in the context of the present invention, the term "solid support" may be understood in the broadest sense as any solid material the antibody may be immobilized on covalently or non-covalently. The solid support may, exemplarily, be a microarray surface (e.g., a glass surface or a hydrogel surface).

In a more preferred embodiment, the solid support is a bead, in particular a magnetic or an affinity chromatography bead.

In the context of the present invention, the terms "bead", "particle", "nanosphere", "precipitate", "colloid" and "aggregate" may be understood interchangeably. The bead may be a magnetic bead, in particular a magnetic mead for cell sorting, or may be an affinity chromatography bead (e.g., a plastic bead (e.g., a polystyrol bead) a glass bead, a silica gel bead, a polysaccharide-based bead (e.g., a sepharose bead)). The bead may also be a microbead or a nanobead.

Further, the solid support may also be a column, a column matrix, a filter, a membrane, in particular a dialysis membrane, a semi-permeable material, or a tubing. In particular the device may be a laboratory device.

According to the second aspect, the present invention refers to a nucleic acid molecule encoding for the antibody according to the present invention.

The term "nucleic acid molecule" may be understood in the broadest sense as deoxyribonucleic acid (DNA) (double stranded DNA (dsDNA), single stranded DNA (ssDNA)), ribonucleic acid (RNA), (single-stranded RNA (ssRNA), double-stranded RNA (dsRNA)), or as a DNA analog such as, e.g., peptide nucleic acid (PNA), morpholino, glycol nucleic acid (GNA), threose nucleic acid (TNA) or methylated DNA. Preferably, the nucleic acid molecule is DNA, in particular double-stranded DNA.

Preferably, the nucleic acid molecule further comprises a start codon, a stop codon and a promoter and potentially an enhancer enabling the translation thereof.

Accordingly, a further aspect of the present invention refers to a vector comprising the nucleic acid molecule encoding for the antibody according to the present invention.

As used in the context of the present invention, the term "vector" may refer to any vehicle enabling the translation of the nucleic acid molecule and expression of the antibody of the present invention in a cell or in a cell-free expression system. The vector may be any vector known in the art such as, e.g., a linear vector, a circular vector, a viral vector or a bacterial vector. Preferably, the vector comprises one or more linear or circular nucleic acid molecule(s) (e.g., a plasmid) or is a viral vector. Optionally, the vector may be a linear expression cassette that may or may not be integrated in the genome of the target cell. However, it may be understood that every other vector may also be used.

In a vector, the nucleic acid molecule encoding for the antibody is typically flanked by starting and stop codons and upstream of this coding region the vector comprises a promoter. Optionally, the vector further comprises one or more enhancer sequences.

As used herein, the term "promoter" may be understood in the broadest sense as a region of DNA that facilitates the transcription of the gene encoding for the antibody. The promoter may be located near the gene, and may be located on the same strand and upstream. Upstream means that it is located towards the 5' region of the sense strand encoding for the antibody. The promoter may be a homologous promoter or a heterologous promoter. The term "expressed" may be understood in the broadest sense as the conversion of genetic information into a polypeptide strand.

In principle, the antibody may be translated and expressed by any means known in the art, i.e., in any cell or cell-free expression system. Preferably, the vector is translated and expressed in a host cell.

Accordingly, a further aspect of the present invention relates to host cell comprising the vector comprising the nucleic acid molecule encoding for the antibody according to the present invention, in particular wherein said host cell is a hybridoma cell.

The host cell in the context of the present invention may be any cell suitable for comprising the vector of the present invention, preferably wherein the host cell is also suitable for translating and expressing the antibody of the present invention.

The host cell may be any cell known in the art. Exemplarily, the host cell may be a prokaryotic or a eukaryotic cell. It may be a bacterial cell (e.g., an *E. coli* cell), an animal cell (e.g., a mammalian cell), a fungal cell (e.g., a yeast cell) or a plant cell. Preferably, the host cell is a bacterial or an animal cell, more preferably a mammalian cell. Even more preferably, the host cell is a B cell lymphocyte or a hybridoma cell. Most preferably, the cell is a B cell lymphocyte or a hybridoma cell.

As used throughout the invention, the terms "hybridoma cell" and "hybrid cell" may be understood interchangeably. Hybridoma cells are generated by means of hybridoma technology which is well-know by those skilled in the art. Herein, a hybridoma cell is generated by fusing an antibody-producing B cell (i.e., a B cell producing antibodies directed against murine XCR1) with a myeloma (B cell cancer) cell that is selected for its ability to grow in tissue culture and for an absence of antibody chain synthesis. Typically, the hybridoma cell will produce monoclonal antibodies. The person skilled in the art will know several methods for generating such hybridoma cell.

Preferably, the hybridoma cell is fusion cell of which at least one of the fused cells is a cell of murine origin, more preferably the hybridoma cell is fusion cell obtained from a fusion of a murine lymphocyte (e.g., a murine B-cell) and a cancer cell (e.g., a cell of a murine myeloma cell line) of murine origin.

Exemplarily, a hybridoma cell may be generated by first inoculating an animal, preferably a mouse, with a tumor cell line (e.g., tumor cell line B16). This inoculation may, exemplarily, be subcutaneous inoculation. The used tumor cell line may secrete growth factors which increase the expansion of murine XCR1-expressing cells such as CD11c⁺ DCs in the animal *in vivo.* Exemplarily, the growth factor may be growth factor Flt3 ligand (March et al., 2000).

Then, the operator will wait for several days such as, e.g., for one, two, three, four, five, six, seven, eight, nine, ten, eleven, twelve, 13, 14, 15, 20, 25, 30, 50 or even more days. Subsequently, the animal may be sacrificed and tissues and/or organs comprising higher amounts of murine XCR1-expressing cells such as CD11c⁺ DCs (e.g., spleen) may be removed from the body.

Optionally, the murine XCR1-expressing cells such as CD11c⁺ DCs may be enriched by any means known for this purpose in the art (e.g., magnetic bead sorting, affinity chromatography, fluorescence activated cell sorting (FACS)). Herein, preferably, antibodies specifically directed to the murine XCR1-expressing cells such as CD11c⁺ DCs (e.g., antibodies binding CD11c) may be used. These antibodies may be polyclonal or monoclonal.

The murine XCR1-expressing cells such as CD11c⁺ DCs may then be used to immunize a homozygous reporter animal (e.g., a B6.XCR1-LacZ mouse) which lacks the murine XCR1 receptor. Here, the DCs will typically be injected into an animal, e.g., intraperitoneally (*i.p.*) or intravenously (*i.v.*). Optionally, this injection will be repeated several times such as, e.g., two times, three times, four times, five times, ten times or even more often. The interval between the single injections may be approximately a day or few days, approximately a week or few weeks, approximately a month or few months. To potentiate the inoculations, the administered compositions may further comprise one or more adjuvants such as, e.g., heat inactivated *Bordetella pertussis* bacteria.

Shortly after the last inoculation such as one, two, three or more days afterwards, the splenocytes of the immunized animal may be extracted and fused with tumor cells such as myeloma cells by means of standard methods.

Alternatively, a host cell may also be generated by immunizing an animal, preferably a mouse, directly with murine XCR1 protein or a peptide sequence of at least eight, nine, ten, eleven or twelve amino acids thereof, a liposome or micelle comprising an murine XCR1 protein or a peptide thereof, a virus presenting murine XCR1 protein or a peptide thereof or a vector enabling the expression of murine XCR1 protein or a peptide thereof in the animal. Then, B-cells or splenocytes are isolated that either may serve as host cells themselves or may be used to generate hybridoma cells as described above.

Alternatively, the host cell may also be a cell in which a vector as described above is inserted by technical means. In this context, the vector may be inserted into the cell by any means known in the art, and may be used in combination with or without transfecting agent(s) (e.g., lithium acetate, polyethylene imine (PEI), fugene, LT-1, JetPEI, transfectamine, lipofectamine, UptiFectin, PromoFectin, GenePORTER, Hilymax, carbon nanofibres, carbon nanotubes cell-penetrating peptides (CPPs), protein transduction domains (PTDs), liposomes, DEAE-dextran, dendrimers), with or without electroporation, or with or without a gene gun, with or without optical transfection with or without gene electrotransfer, with or without impalefection, with or without magnetofection, and/or with or without magnet assisted transfection. This biotechnological method is particularly preferred when using a bacterial cell or heterologous mammalian or insect cell as host cell.

Optionally, the host cells, independent on how it is generated, may be screened by one or more immunological method(s) such as, e.g., flow cytometry, enzyme-linked immunosorbent assay (ELISA), Western Blot.

The antibody-expressing host cells producing antibodies binding to CD8⁺ DCs may be recovered. These recovered cells may, optionally, be tested using reporter animals or cell cultures such as, e.g., cells comprising a murine XCR1-LacZ reporter gene (e.g., obtained from a homozygous B6-XCR1-LacZ mouse). Further, optionally, the activity of the antibody on cells in the presence ofXCL1 (e.g., effect on calcium influx) may be tested by any means known in the art.

The host cells may be cultured at conditions suitable for these cells to survive and, preferably, to proliferate. The host cell may also be stored in frozen, deep-frozen, freeze-dried and/or dry state depending on the biological nature of the host cell.

In order to generate the antibody, the host cells may be cultured at conditions enabling the generation of the antibody. Optionally, the antibody may then be recovered from the culture broth.

In a further aspect, the present invention refers to a method for producing the antibody according to the present invention, said method comprising:
(i) culturing a host cell according to the present invention under conditions that allow said host cell to express said antibody;
(ii) recovering said antibody from said culture.

As used herein, culturing a host cell may be adjusted to the host cells individually. The person skilled in the art will know the standard growth conditions for particular bacteria and particular eukaryotic cells from standard text books.

The term "recovering said antibody" as used herein may be understood in the broadest sense as any means for obtaining the antibody from the culture. As the host cells will typically be cultured in a culture broth, also designated as culture medium, the antibody is present in a mixture comprising several salts, sugars, buffer agents, amino acids, growth factors and cells. Recovering of the antibody means that an antibody- comprising part of this mixture is taken from the cell culture. Preferably, recovering the antibody may further comprise purifying the antibody from the culture broth. Then, the antibody concentration is increased. It will, however, be understood that also an isolated antibody may optionally be dissolved in a liquid medium comprising further components.

The antibody may, optionally, also be isolated from the cell culture. Then, the antibody concentration is enriched up to at least 50 %(w/w), at least 60 %(w/w), at least 70 %(w/w), at least 80 %(w/w), at least 90 %(w/w), at least 95 %(w/w), or even 100 %(w/w) of the total antibody content. Preferably, an isolated antibody may constitute for at least 20 %(w/w), at least 40 %(w/w), at least 50 %(w/w), at least 60 %(w/w), at least 70 %(w/w), at least 80 %(w/w), at least 90 %(w/w) or even 100 %(w/w) of the total protein content. It will, however, be understood that also an isolated antibody may optionally be dissolved in a liquid medium comprising further components.

The recovered antibody may or may not be conjugated to at least one labeling moiety and/or at least one solid support by any chemical and/or biological means known for such conjugations known in the art. The person skilled in the art will know several conjugation methods such as, e.g., the formation of amide bonds by means of an active ester e.g., succinimidyl ester) or the conjugation of a thiol moiety with a maleimide.

In general, the antibody is also usable for any means an antibody may be used for, in particular for detecting cells and separating cells.

Therefore, according to a further aspect, the present invention refers to a use of the antibody according to the present invention for
(i) detecting murine XCR1⁺ cells, in particular by means of cytometry, immunohistology and/or molecular imaging; and/or
(ii) separating murine XCR1⁺ cells from other cells, in particular by means of cell sorting.

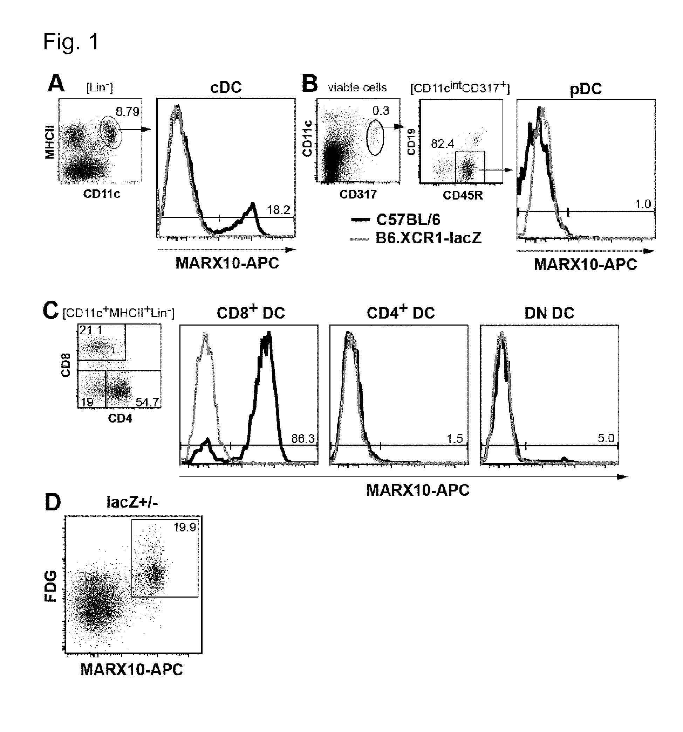

As used herein, the term "detecting murine XCR1⁺ cells" may be understood in the broadest sense as the determination or assessment of the localization, concentration and/or number of murine XCR1⁺ cells. As also shown in the examples below, the antibody of the present invention may be used to identify murine XCR1⁺ cells by flow cytometry (as shown in Fig. 1), by immunohistology or any other standard technique. Antibodies to murine XCR1 can be used to separate murine XCR1⁺ cells from other cells by standard separation techniques (e.g. magnetic bead sorting).

Preferably, detecting murine XCR1⁺ cells is performed by cytometry, immunohistology and/or molecular imaging.

As used herein, the terms "cytometry" is particularly flow cytometry, a well-known method known in the art. In the context of the present invention, preferably a sample comprising a cell suspension is contacted with a fluorescently labeled antibody of the present invention and administered to a flow cytometer. This method enables the quantification of the concentration of murine XCR1⁺ cells in the sample, the ration between murine XCR1⁺ cells and murine XCR1⁻ cells and to assess the size and shape of the cells.

In the context of the present invention, the terms "immunohistology" and "immunostaining" may be understood in the broadest sense as any method based on the staining of cells and/or tissues outside the body. Most typically, the antibody of the present invention may serve as a primary antibody which binds to its molecular target murine XCR1 on the cells and/or tissues. In a further subsequent step, the Fc part may then be bound by a secondary antibody that bears at least a labeling moiety as described above, such as, e.g., fluorescent dye, a non-fluorescent dye an enzyme converting a dye precursor into a dye and/or a metal bead. Immunohistology may be performed with living cells or tissues or with fixed cells and/or tissues. The readout is typically performed by means of microscopy (e.g., bright field microscopy (light microscopy), fluorescence microscopy, laser scanning microscopy, two-photon fluorescence microscopy, electron microscopy).

Alternatively or additionally, the antibody of the present invention itself may be conjugated with at least one labeling moiety as described herein, e.g., a fluorescent dye, a non-fluorescent dye an enzyme converting a dye precursor into a dye, a rare-earth element and/or a metal bead.

Exemplarily, molecular imaging may be Fluorescence Molecular Imaging (FMI), in particular Fluorescence Molecular Tomography (FMT), Magnetic Resonance Imaging (MRI), Optical Imaging, Single Photon Emission Computed Tomography (SPECT), Positron Emission Tomography (PET), Ultrasound or Computer Tomography (CT), fluorescence microscopy, laser scanning microscopy, two-photon microscopy, fluorescence energy transfer (FRET) based methods, fluorescence correlation spectroscopy (FCS), fluorescence cross-correlation spectroscopy (FCCS), electron microscopy or combinations of two or more thereof. These techniques are well-known to those skilled in the art.

Moreover, the present invention may be used in any other immunological method known in the art, such as e.g., fluorescence activated cell sorting (FACS), by Western Blot, by ELISA.

In all the methods described herein, the antibody of the present invention may serve as a primary antibody and/or as a secondary antibody. A primary antibody typically binds to its molecular target murine XCR1 on the cells and/or tissues. In a further subsequent step, the Fc part may then be bound by a secondary antibody that bears at least a labeling moiety as described above, such as, e.g., fluorescent dye, a non-fluorescent dye an enzyme converting a dye precursor into a dye and/or a metal bead.

Alternatively or additionally, the antibody of the present invention itself may be conjugated with at least one labeling moiety as described herein.

It will be apparent that all embodiments described herein may also form part of a commercial kit.

Accordingly, according to a further aspect of the present invention refers to a kit for detecting murine XCR1⁺ cells, said kit comprising the antibody according to the present invention, a nucleic acid according to the present invention, a vector according to the present invention and/or a host cell according to the present invention; and preferably further comprising a user manual.

Such user manual may, exemplarily, comprise information on how to use the antibody in the detection methods described above.

According to a still further aspect, the present invention refers to a kit for separating murine XCR1⁺ cells from other cells, said kit comprising the antibody according to the present invention, a nucleic acid according to the present invention, a vector according to the present invention and/or a host cell according to the present invention; and preferably further comprising and a user manual.

Cell separation may be performed by any means known in the art, such as, e.g., by staining the cells with the antibody of the present invention that is conjugated to a labeling moiety or the antibody of the present invention that serves as a primary antibody and binding a secondary antibody which is labeled accordingly to its Fc part and subsequent cell sorting by a cell sorter. Particularly preferred such cell sorting is based on fluorescence activated cell sorting (FACS) wherein the antibody of the present invention and/or a secondary antibody binding thereto is fluorescently labeled. Cell separation may also be performed by means of beads coated with the antibody of the present invention, in particular wherein the beads are magnetic beads. Cell separation may also be performed by means of affinity chromatography, wherein the antibody of the present invention is immobilized on the chromatographic matrix.

Herein, the user manual may, exemplarily, comprise information on how to use the antibody in separation.

Moreover, a kit according the present invention may optionally further comprise one or more carrier(s). Exemplarily, said carrier may be a solvent such as, e.g., water, dimethyl sulfoxide (DMSO), ethanol, vegetable oil, paraffin oil or combinations thereof. Furthermore, a carrier may contain one or more detergents, one or more foaming agents (e.g., sodium lauryl sulfate (SLS)/ sodium doceyl sulfate (SDS)), one or more colouring agents (e.g., TiO₂, food colouring), one or more vitamins, one or more salts (e.g., sodium, potassium, calcium, zinc salts), one or more humectants (e.g., sorbitol, glycerol, mannitol, propylenglycol, polydextrose), one or more enzymes, one or more preserving agents (e.g., benzoic acid, methylparabene), one or more texturing agents (e.g., carboxymethyl cellulose (CMC), polyethylene glycol (PEG), sorbitol), one or more emulsifiers , one or more bulking agents, one or more glacing agents, one or more separating agents, one or more antioxidants, one or more herbal and plant extracts, one or more stabilizing agents, one or more polymers (e.g., hydroxypropyl methacrylamide (HPMA), polyethylene imine (PEI), carboxymethyl cellulose (CMC), polyethylene glycol (PEG)), one or more uptake mediators (e.g., polyethylene imine (PEI), dimethyl sulfoxide (DMSO), a cell-penetrating peptide (CPP), a protein transduction domain (PTD), an antimicrobial peptide, etc.) one or more antibodies, one or more sweeteners (e.g., sucrose, acesulfam K, saccharin Na, stevia), one or more counterstain dyes (e.g., fluorescein, fluorescein derivatives, Cy dyes, an Alexa Fluor dyes, S dyes, rhodamine, quantum dots, etc.), one or more homeopathic ingredients one or more gustatory substances and/or one or more fragrances.

If not otherwise defined, all numerical specifications are to be considered to be approximate values allowing typical deviations of several percent. The present invention further also includes the salts of any of the compounds described herein.

### BRIEF DESCRIPTION OF THE DRAWING

**Figure 1** shows mAb MARX10 specifically recognizing murine XCR1 which is expressed on subsets of cDCs. Splenocytes from C57BL/6 (black lines) or homozygous B6.XCR1-lacZ mice lacking the murine XCR1 receptor (gray lines) were stained with mAb MARX10 and gated on (A) conventional DCs (cDC), (B) plasmacytoid DCs (pDC), or (C) the CD8⁺, CD4⁺, or double-negative (DN) cDC subsets. MAb MARX10 stained 18 % of all cDCs, 86 % of CD8⁺ cDCs and 5 % DN DCs, no staining was observed with pDCs. In homozygous B6.XCR1-lacZ mice no DC staining was observed (gray lines), confirming the murine XCR1-specificity of mAb MARX10. (D) Double staining for the lacZ reporter in B6.XCR1-LacZ heterozygous mice using fluorescein-di-β-D-galactopyranoside (FDG) according to the manufacturer (Invitrogen) and murine XCR1 using mAb MARX10.

### EXAMPLES

The following examples as well as the accompanying figures are intended to provide illustrative embodiments of the present invention described and claimed herein. These examples are not intended to provide any limitation on the scope of the invented subject-matter.

### Generation of an antibody binding to murine XCR1

Wild type C57BL/6 mice were subcutaneously implanted with the tumor line B16 (1x10⁶ in 100 µl), which secretes the growth factor Flt3 ligand (Mach et al., 2000). Exposure to Flt3 ligand resulted in a massive expansion of the murine XCR1-expressing CD11c⁺ DC *in vivo.*

After 12 days, spleens were removed from the inoculated animals and CD11c⁺DC were enriched by positive magnetic sorting using an antibody to CD 11c (Miltenyi Biotech, Bergisch-Gladbach, Germany). The CD11c-enriched DC populations were then used to immunize homozygous reporter mice (B6.XCR1-LacZ), which lack the murine XCR1-receptor.

For immunization, the animals were injected intraperitoneally every 3 weeks (4 times in succession) with 30x10⁶ CD11c-enriched cells in combination with heat-inactivated *Bordetella pertussis* bacteria (Behring-Chiron, Marburg, Germany). One day after the last boost, splenocytes of the immunized mice were fused with the myeloma cell line P3X63Ag8.653 (ATCC) according to standard methods.

### Screening of the host cells

Screening of the resulting hybridomas for specific detection of murine XCR1 was by flow cytometry.

For flow cytometry, surface staining reagents were titrated for optimal signal-to-noise ratio. To block unspecific binding to Fc-receptors, cells were pre-incubated with 100 µg/ml 2.4G2 mAb. Standard staining with was in PBS, 0.25 % BSA, 0.1 % NaN₃ for 20 min on ice. Data were acquired on a LSR II flow cytometer (BD Biosciences), and analyzed using FlowJo (Tree Star Inc.) (Domer et al., 2009).

All antibodies recognizing CD8⁺ cDCs from wildtype (wt) animals were regarded as candidates.

In the next step, these candidate mAb were tested on splenic DC from homozygous B6.XCR1-LacZ reporter mice, which lack the murine XCR1 receptor. One mAb, designated MARX10 (IgG2b) fulfilled the screening criteria in that it stained CD8⁺ cDCs from C57BL/6 wild type animals, but not CD8⁺ cDCs from homozygous B6.XCR1-LacZ reporter mice (Fig. 1A-C).

Specificity of MARX10 for murine XCR1 was further verified by staining of cDCs from heterozygous B6.XCR1-LacZ mice which do express murine XCR1 on the cell surface; here the signal obtained with mAb MARX10 was correlated with expression of the lacZ-reporter (Fig. 1D). Mab MARX10 did not block chemotaxis of murine XCR1⁺ DCs to the murine chemokine ligand XCL1. MAb MARX10 did not show any staining of populations or tissues other than cDCs in the mouse.

### REFERENCES

Bachem, A., Güttler, S., Hartung, E., Ebstein, F., Schaefer, M., Tannert, A., Salama, A., Movassaghi, K., Opitz, C., Mages, H. W., Henn, V., Kloetzel, P. M., Gurka, S. and Kroczek, R. A. (2010). Superior antigen cross-presentation and XCR1 expression define human CD11c+CD141+ cells as homologues of mouse CD8+ dendritic cells. J. Exp. Med. 207, 1273-1281.
Crozat, K., Guiton, R., Contreras, V., Feuillet, V., Dutertre, C. A., Ventre, E., Vu Manh, T. P., Baranek, T., Storset, A. K., Marvel, J., Boudinot, P., Hosmalin, A., Schwartz-Comil, I. and Dalod, M. (2010). The XC chemokine receptor 1 is a conserved selective marker of mammalian cells homologous to mouse CD8α+ dendritic cells. J. Exp. Med. 207, 1283-1292.
Domer, B. G., Domer, M. B., Zhou, X., Opitz, C., Mora, A., Güttler, S., Hutloff, A., Mages, H. W., Ranke, K., Schaefer, M., Jack, R. S., Henn, V. and Kroczek, R. A. (2009). Selective expression of the chemokine receptor XCR1 on cross-presenting dendritic cells determines cooperation with CD8+ T cells. Immunity 31, 823-833.
Hashimoto, D., J. Miller, and M. Merad (2011). Dendritic cell and macrophage heterogeneity in vivo. Immunity 35, 323-335
Kelner, G. S., Kennedy, J., Bacon, K. B., Kleyensteuber, S., Largaespada, D. A., Jenkins, N. A., Copeland, N. G., Bazan, J. F., Moore, K. W., Schall, T. J. and Zlotnik, A. (1994). Lymphotactin: a cytokine that represents a new class of chemokine. Science 266, 1395-1399.
Lefranc M.P. et al., (2003) IMGT unique numbering for immunoglobulin and T cell receptor variable domains and Ig superfamily V-like domains. Dev Comp Immunol., 27, 55-77.
Mach, N., S. Gillessen, S.B. Wilson, C. Sheehan, M. Mihm, and G. Dranoff. 2000. Differences in dendritic cells stimulated in vivo by tumors engineered to secrete granulocyte-macrophage colony-stimulating factor or Flt3-ligand. Cancer Res. 60, 3239-3246.
Müller, S., Dorner, B., Korthäuer, U., Mages, H. W., D'Apuzzo, M., Senger, G. and Kroczek, R. A. (1995). Cloning of ATAC, an activation-induced, chemokine-related molecule exclusively expressed in CD8+ T lymphocytes. Eur. J. Immunol. 25, 1744-1748.
Yoshida, T., Imai, T., Kakizaki, M., Nishimura, M. and Yoshie, O. (1995). Molecular cloning of a novel C or gamma type chemokine, SCM-1. FEBS Lett. 360, 155-159.
Yoshida, T., Imai, T., Kakizaki, M., Nishimura, M., Takagi, S. and Yoshie, O. (1998). Identification of single C motif-1/lymphotactin receptor XCR1. J. Biol. Chem. 273, 16551-16554.
Yoshida, T., Izawa, D., Nakayama, T., Nakahara, K., Kakizaki, M., Imai, T., Suzuki, R., Miyasaka, M. and Yoshie, O. (1999). Molecular cloning of mXCR1, the murine SCM 1/ lymphotactin receptor. FEBS Lett. 458, 37-40.

## Claims

1. An antibody binding to murine XCR1.

2. The antibody according to claim 1, wherein said antibody is a monoclonal antibody.

3. The antibody according to claim 1 or 2, wherein said antibody is specific for dendritic cells, in particular murine dendritic cells.

4. The antibody according to any one of claims 1 to 3, wherein said antibody does not block chemotaxis of XCR1⁺ dendritic cells to the murine chemokine ligand XCL1.

5. The antibody according to any one of claims 1 to 4, wherein said antibody is a murine antibody.

6. The antibody according to any one of claims 1 to 5, wherein said antibody is conjugated with at least one labeling moiety.

7. The antibody according to any one of claims 1 to 6, wherein said antibody is conjugated with at least one solid support,

8. The antibody according to claim 7, wherein the solid support is a bead, in particular a magnetic or an affinity chromatography bead.

9. A nucleic acid molecule encoding for the antibody according to any one of claims 1 to 6.

10. A vector comprising the nucleic acid molecule according to claim 9.

11. A host cell comprising the vector according to claim 10, in particular wherein said host cell is a hybridoma cell.

12. A method for producing the antibody according to any one of claim 1 to 6, said method comprising:
(i) culturing a host cell according to claim 11 under conditions that allow said host cell to express said antibody;
(ii) recovering said antibody from said culture.

13. Use of the antibody according to any one of claims 1 to 8 for
(i) detecting murine XCR1⁺ cells, in particular by means of cytometry, immunohistology; and/or
(ii) separating murine XCR1⁺ cells from other cells, in particular by means of cell sorting.

14. A kit for detecting murine XCR1⁺ cells, said kit comprising the antibody according to any one of claims 1 to 8, a nucleic acid according to claim 9, a vector according to claim 10 and/or a host cell according to claim 11; and further comprising a user manual.

15. A kit for separating murine XCR1⁺ cells from other cells, said kit comprising the antibody according to any one of claims 1 to 8, a nucleic acid according to claim 9, a vector according to claim 10 and/or a host cell according to claim 11; and further comprising a user manual.
